# FASCICULE DE BREVET EUROPEEN

(11) **EP 0 609 760 B1**
(45) Date de publication et mention de la délivrance du brevet: **29.07.1998**
(21) Numéro de dépôt: 94101104.1
(22) Date de dépôt: 26.01.1994
(51) Int. Cl.: G01N 27/28, C12Q 1/00

(54) **Système de mesures électrochimiques à capteur multizones, et son application au dosage du glucose**
Elektrochemisches System mit mehrzonigem Sensorelement zur Bestimmung von Glukose
Electrochemical measurement system with snap-off sensor zones for the determination of glucose

(30) Priorité: 04.02.1993 FR 9301331
(43) Date de publication de la demande: 10.08.1994
(73) Titulaire: ASULAB S.A., CH-2501 Bienne (CH)
(72) Inventeur: Saurer, Eric, CH-2022 Bevaix (CH); Frenkel, Erik Jan, CH-2000 Neuchâtel (CH); Randin, Jean-Paul, CH-2016 Cortaillod (CH); Hoffmann, Eric, CH-2563 Ipsach (CH)
(74) Mandataire: Thérond, Gérard Raymond

(56) Documents cités:
- EP-A- 0 373 413
- WO-A-90/10861
- WO-A-91/00998
- WO-A-92/14836
- WO-A-92/21961
- US-A- 4 952 266

## Description

La présente invention a pour objet un système de mesures électrochimiques constitué par un appareil de mesures destiné à recevoir un capteur de petite dimension, à multizones actives permettant d'effectuer plusieurs mesures successives d'une même donnée au moyen d'un unique capteur introduit dans l'appareil de mesure.

De nombreuses techniques électrochimiques ont été développées pour détecter au moyen d'électrodes et d'un circuit électrique ou électronique approprié les caractéristiques physico-chimiques d'un milieu, ou pour déterminer les concentrations de substances présentes dans des solutions, des effluents, ou des fluides d'origine naturelle biochimique ou biologique. Ces techniques font généralement appel à des mesures conductimétriques, voltamétriques, ampérométriques, coulométriques ou polarographiques. Selon les mesures qu'on souhaite effectuer, ces techniques électrochimiques peuvent en outre faire intervenir un réactif ajouté à la solution, ou confiné au voisinage d'une ou de plusieurs électrodes.

Par exemple, pour le dosage du glucose présent dans le sang, le réactif comprendra au moins une enzyme spécifique du glucose.

Les systèmes pour effectuer de telles mesures en laboratoire sont connus depuis longtemps; ils sont généralement encombrants, difficilement transportables, coûteux et exigent de plus les compétences d'un spécialiste pour leur mise en oeuvre, ainsi qu'un entretien et un nettoyage minutieux après chaque mesure.

Depuis quelques décennies la tendance a donc été à rechercher des systèmes plus petits, voire portables, peu coûteux, et facilement utilisables par quelqu'un ne possédant pas de compétences particulières.

Très tôt, il est donc apparu utile de pouvoir séparer la partie du système permettant la saisie des données, c'est-à-dire le dispositif supportant les électrodes, de l'appareil permettant de traiter ces données et de les afficher. Dès lors qu'on a envisagé de séparer les électrodes servant à la saisie des données du reste de l'appareil de mesure, on a tout naturellement été conduit à réunir physiquement lesdites électrodes sur un même support isolant pour maintenir leur disposition spatiale; un tel dispositif constitué par un support comportant des électrodes connectables à un appareil de mesure est désigné sous le terme général de "capteur", et dans le cadre de la présente invention de "capteur électrochimique". En fonction du choix des matériaux et des techniques de production de masse adoptés pour sa fabrication, il a également été possible de réduire considérablement le coût unitaire d'un capteur au point d'envisager son remplacement après chaque mesure.

La réduction de l'encombrement de l'appareil de mesure lui-même a tout naturellement résulté de la miniaturisation rendue possible par les progrès de l'électronique; l'appareil de mesure selon l'invention aura avantageusement les dimensions d'un étui portable.

Concernant le capteur électrochimique lui-même, le brevet DE 2 127 142 décrit, par exemple, un dispositif dans lequel deux électrodes sont disposées à plat par la technique de déposition dite en couche mince, sur une plaquette isolante de petite dimension.

Le brevet US 4 076 596 décrit aussi un capteur à oxygène dans lequel l'anode et la cathode sont déposées sur un substrat isolant en matière plastique par une technique dite en couche épaisse ou en couche mince. Selon une variante, ce capteur comporte également une troisième électrode servant de référence.

Un dispositif à trois électrodes est également décrit dans le brevet JP 61.294351 qui comporte, en outre, à titre de réactif déposé sur les électrodes, un mélange titre de réactif déposé sur les électrodes, un mélange d'enzyme oxydoréductase et un médiateur permettant d'effectuer des mesures de taux de glucose par une mesure électrochimique.

D'autres capteurs ont également été décrits comme comportant plus de trois électrodes. Le brevet EP 0 471 986 décrit notamment un capteur à quatre électrodes, deux électrodes servant à la détection de la présence de l'échantillon à analyser, les deux autres servant à effectuer la mesure, elle-même, par ampérométrie.

Certaines autres variantes ont conduit plusieurs auteurs à désigner des capteurs sous le nom de "multicapteur". Cette désignation "multicapteur" a par exemple été utilisée lorsque plusieurs capteurs discrets sont disposés sur une même plaque isolante pour effectuer, simultanément ou non, une série de mesures, chaque capteur discret ayant son propre système de connexion à un appareil de mesure. Un dispositif de ce type est par exemple décrit dans le brevet EP 0 170 375.

Dans le document WO 90/10861, il est prévu de pouvoir effectuer plusieurs mesures avec un unique capteur formé d'électrodes noyées dans un corps isolant en forme de bâtonnet et dont on forme la zone active de mesure sur sa section droite par élimination d'une position de son extrémité. Selon une variante décrite dans le document WO 92/21961 le capteur peut également avoir une forme de languette.

Les capteurs correspondant aux types qui viennent d'être cités sont généralement de petites dimensions et sont réalisés dans des matériaux et par des techniques permettant de réduire le coût unitaire, de sorte qu'ils puissent être jetés après quelques usages, voire après le premier usage lorsqu'ils incorporent un réactif consommable. Lorsqu'ils sont qualifiés de "multicapteurs", ces capteurs, soit possèdent des jeux l'électrodes remplissant des fonctions différentes pour effectuer un dosage unique sur un échantillon (EP 0 471 986), soit constituent une série de capteurs discrets individuellement connectables (EP 0 170 375), soit sont formés au fur et à mesure des besoins en bout de capteur par sectionnement d'un matériau isolant enrobant totalement des collecteurs de courant dont les sections droites formeront les électrodes (WO 90/10861, WO 92/21961).

Le capteur selon l'invention est au contraire constitué par un ensemble de zones actives, généralement toutes identiques, disposées sur un substrat isolant en forme de bande muni d'au moins deux collecteurs de courant en matériaux conducteurs électriquement isolés, lesdites zones actives devant être détachées les unes après les autres après chaque usage pour effectuer la mesure suivante. Un tel capteur selon l'invention diffère des "multicapteurs" antérieurement connus et peut être désigné comme étant un "capteur à multizones actives séquentiellement jetables". Il permet en effet, après avoir introduit et connecté une bande de longueur déterminée dans un appareil de mesure, d'effectuer successivement plusieurs mesures d'une même caractéristique physico-chimique ou de la concentration d'un même constituant dans l'échantillon de solution déposé successivement sur une nouvelle zone de mesure du capteur, la zone précédemment utilisée ayant été éliminée.

Dans le capteur à multizones actives séquentiellement jetables selon l'invention les collecteurs de courant sont situés sur une même face de la bande ou sur les faces opposées, chaque collecteur ayant au moins une portion constituant un moyen de contact avec l'appareil de mesure et au moins une autre portion traversant successivement au moins deux zones actives, délimitées par des fenêtres ménagées dans une ou plusieurs bandes de recouvrement, dans lesquelles les parties apparentes des collecteurs de courant constituent les électrodes. La bande peut en outre être pourvue de moyens de positionnement et/ou de sectionnement après usage.

Les zones de contact des collecteurs de courant en matériaux conducteurs se trouvent situées, soit à une extrémité de la bande, soit le long d'un ou des deux bords de la bande, soit réparties entre une extrémité et les bords de la bande. Le nombre de zones de mesure sur une unité de bande donnée introduite dans l'appareil de mesure dépendra, pour un appareil de mesure donné, d'une part de la dimension physique des zones actives, d'autre part de la distance entre celles-ci, cette distance étant imposée, entre autre, par la facilité d'accès à la zone de mesure positionnée à l'extérieur de l'appareil en vue de la saisie de l'échantillon dont on veut mesurer les caractéristiques.

En fonction de la fréquence de mesures souhaitée dans un intervalle de temps donné, la bande est configurée sous forme d'une barrette ou sous la forme d'un rouleau. La longueur maximum de la bande est fonction du délai de conservation des réactifs à l'air libre et de la fréquence des dosages.

Compte-tenu des petites dimensions de l'appareil de mesure lui-même, le capteur a une épaisseur comprise entre 0,05 et 0,5 mm et une largeur comprise entre 6 et 12 mm; lorsqu'il se présente sous forme d'une barrette, le capteur a une longueur comprise de préférence entre 10 et 20 cm. Après introduction du capteur dans l'appareil de mesure et connexion au dispositif électronique de mesure, les zones actives sont rendues accessibles séquentiellement, chaque zone active étant identifiée et mise en place par des moyens appropriés intégrés en vue de la mesure elle-même. D'autres moyens également intégrés permettront, à la fin de la mesure, l'élimination de la zone active utilisée et ultérieurement la mise en place d'une nouvelle zone active. Le capteur selon l'invention est utilisable pour tout type de mesure électrochimique, mais il est particulièrement approprié pour les mesures effectuées par l'intermédiaire d'un réactif consommable.

L'appareil de mesure est composé d'un boîtier à l'intérieur duquel est logé un circuit électronique et des dispositifs mécaniques.

Une source d'énergie, située à l'intérieur dudit boîtier permet d'imposer entre les contacts recevant un capteur un courant électrique variable ou non, ou une tension électrique variable ou non au moyen d'un circuit électronique approprié, ledit circuit étant également destiné d'une part à mesurer respectivement la tension ou le courant aux bornes des contacts et d'autre part à traiter ce signal électrique en vue de l'affichage de la valeur représentative de la caractéristique physicochimique mesurée. Le circuit électronique peut en outre comporter un thermomètre permettant de corriger les effets d'une variation de température sur la mesure; il peut également être pourvu d'une horloge et d'une alarme avertissant l'utilisateur des instants où doivent être effectuées les mesures. L'appareil de mesure comporte également un dispositif permettant de connecter électriquement le capteur introduit dans le boîtier au circuit électronique pendant la durée de toute une série de mesures.

Le boîtier comporte une ouverture permettant l'introduction d'un capteur; il peut également comporter deux ouvertures, une étant destinée au chargement du capteur, l'autre étant destinée à rendre accessible séquentiellement les zones de mesure. Le boîtier contient aussi un dispositif d'entraînement permettant de déplacer et de positionner le capteur à l'intérieur du boîtier par des moyens mécaniques ou électromécaniques. Ce dispositif coopère avec le capteur par exemple au moyen d'encoches pratiquées dans l'épaisseur d'au moins un bord de la bande support du capteur, ou de rainures dans une face du capteur selon des lignes perpendiculaires à l'axe median du capteur. Le boîtier incorpore enfin, un dispositif permettant d'éliminer la zone de mesure utilisée.

A titre d'exemple non limitatif la description ci-après se rapportera à un système de mesures électrochimiques constitué par un appareil de mesure ampérométrique destiné à recevoir un capteur pour la mesure de la concentration d'un composant en solution lorsqu'une enzyme oxydoréductase spécifique dudit composant catalyse une réaction d'oxydoréduction et qu'un médiateur facilite le transfert des électrons entre ladite enzyme et une électrode de travail. Plus particulièrement, l'invention concerne, à titre d'exemple, un capteur du type précédent pour le dosage du glucose dans le sang dans lequel le réactif consommable comporte notamment à titre d'enzyme spécifique la glucose-oxydase (GOD) et un médiateur choisi parmi les complexes d'un métal de transition avec au moins un ligand bipyridine, terpyridine ou phénantroline substitué par au moins un groupe donneur d'électrons. En pareille hypothèse l'unité de bande comportera de préférence autant de zones de mesure que de dosages de glucose prescrits par jour ou par semaine.

L'invention fournit aussi un capteur dont le réactif déposé dans toutes les zones de mesures provient nécessairement d'un même lot de fabrication, ce qui permet d'effectuer, dans une unité de temps donnée, une série de mesures ayant une très grande précision relative, voire une très grande précision absolue pour autant que la première zone de mesure soit utilisée avec une solution étalon permettant une calibration de l'appareil de mesure.

L'invention sera mieux comprise en référence à un système de mesure comportant un capteur pour le dosage par méthode ampérométrique du glucose dans le sang en présence d'un réactif contenant notamment un glucose oxydase (GOD) et un médiateur. Un tel système correspond de façon non limitative aux différents modes de réalisation tels que ceux représentés dans les dessins annexés dans lesquels :
- la figure 1 représente une vue en perspective d'un premier mode de réalisation d'un capteur selon l'invention;
- la figure 2 représente une vue éclatée de la figure 1;
- la figure 3 représente une vue en coupe de la figure 1 selon la ligne III-III;
- la figure 4 représente une vue en perspective d'un deuxième mode de réalisation d'un capteur selon l'invention;
- la figure 5 représente une vue en coupe de la figure 4 selon la ligne V-V;
- la figure 6 représente une vue en perspective d'un troisième mode de réalisation d'un capteur selon l'invention;
- la figure 7 représente une vue en perspective agrandie de l'extrémité d'un capteur selon la figure 6;
- la figure 8 représente une vue en coupe de la figure 6 selon la ligne VIII-VIII;
- la figure 9 représente une vue en coupe d'une variante du capteur selon la figure 6, selon une même ligne VIII-VIII;
- la figure 10 représente une vue en perspective d'un quatrième mode de réalisation d'un capteur selon l'invention;
- la figure 11 représente une vue en coupe de la figure 10 selon la ligne XI-XI;
- la figure 12 représente une vue de face en perspective partiellement arrachée, de l'appareil de mesure;
- la figure 13 représente une vue arrière en perspective, partiellement arrachée de l'appareil de mesure;
- la figure 14 représente une vue en perspective agrandie du dispositif de connexion du capteur dans l'appareil de mesure.

Dans toutes les représentations en coupe les proportions entre les épaisseurs des différentes couches de matériaux du capteur et sa largeur ne sont pas respectées de façon à montrer plus clairement l'agencement des différentes couches constituant ledit capteur.

Dans toutes les figures, la même référence désigne une partie similaire du boîtier ou du capteur, quelque soit le mode de réalisation.

Dans les figures ayant des éléments répétitifs, seul un de ces éléments comporte des références.

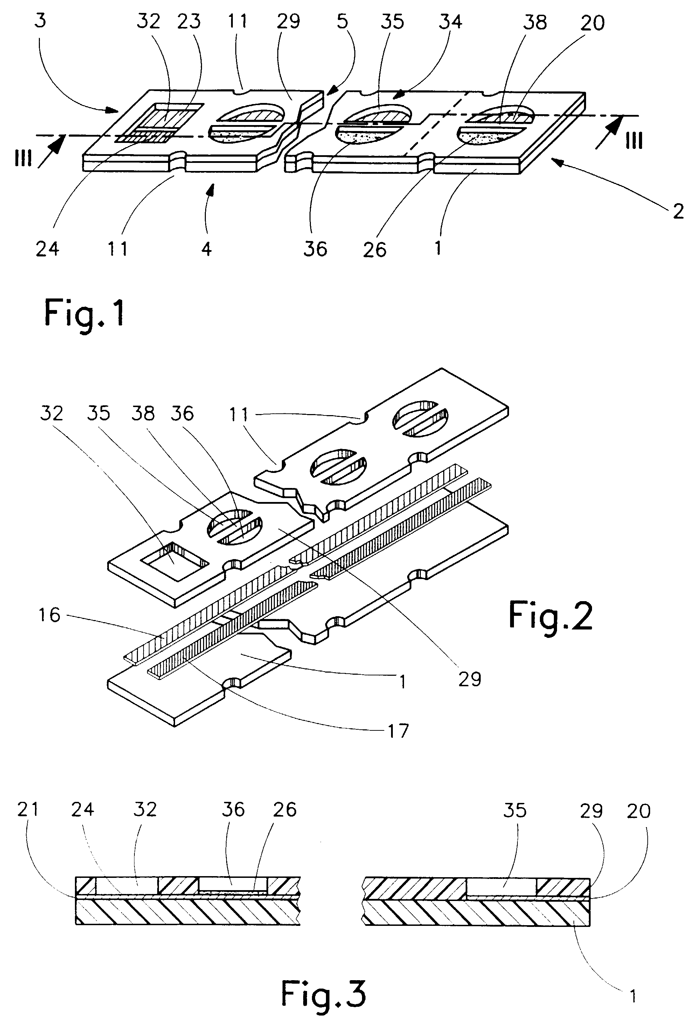

Selon un premier mode de réalisation, représenté aux figures 1 à 3, le capteur est constitué par une bande support en matériau isolant 1, étroite, - de l'ordre de 6 à 12 mm -, et de faible épaisseur, - de l'ordre de 0,05 à 0,5 mm -. A titre de matériau isolant, on utilisera par exemple une matière plastique souple tel qu'un polyéthylène téréphtalate (PET), commercialisé par exemple sous les marques de Hostaphan® ou Mylar®. Sur toute la longueur de cette bande sont disposés deux collecteurs de courant 16, 17, représentés pour simplifier sous forme de fines bandes parallèles aux bords 4 et 5 du support 1 et séparées par un très petit espace 38. Ces collecteurs de courant constitueront respectivement les électrodes de travail et de référence respectivement. Ils sont réalisés en un matériau conducteur résistant à la corrosion par des techniques connues dites de déposition en couche mince ou épaisse, ou par laminage de films revêtus desdits matériaux conducteurs.

A titre d'exemple de matériaux conducteurs résistant à la corrosion, employés pour l'électrode de travail, on utilise des métaux tels que l'or, le platine, l'argent, le nickel, le palladium ou le titane, ainsi que le carbone. L'électrode de référence la plus souvent utilisée est constituée par de l'argent recouvert d'une couche de chlorure d'argent. Les électrodes portées par la plaquette sont ensuite recouvertes par un film isolant 29 d'épaisseur égale ou inférieure à celle de la bande support 1; le matériau constituant le film 29 peut être identique à celui de la bande support 1, différent ou composite, tel qu'une bande en PET revêtue d'une couche d'un matériau thermofusible, par exemple de polyéthylène, ou d'une colle.

Préalablement à son application, le film protecteur 29, qui recouvre la totalité de la surface de la bande support 1 est pourvu de fenêtres 32, 34. La fenêtre 32 située près de l'extrémité 3 de la bande est de forme rectangulaire et laisse apparaître les portions de conducteur de courant 23,24 destinées à être connectées à l'appareil de mesure. Les fenêtres 34, régulièrement espacées, délimitent les zones actives séquentiellement jetables, de forme circulaire, où seront déposés les échantillons à mesurer. Les fenêtres 34 peuvent avoir toute autre forme appropriée, telle qu'une forme elliptique ou rectangulaire. Chaque zone peut être divisée par une étroite bande de matière ayant pour largeur l'espacement 38 entre les électrodes, délimitant ainsi deux secteurs en demi-lune 35,36 laissant chacun apparaître une portion d'électrode 20, 21. Le réactif 26 contenant notamment la glucose-oxydase (GOD) et un médiateur est ensuite déposé sur les portions d'une électrode situées dans chaque zone en demi-lune par les techniques connues de pipetage, sérigraphie ou tamponnage. Sur les bords 4, 5 de la bande 1 des encoches 11 sont prévues pour assujettir le capteur au dispositif d'avance intégré dans l'appareil de mesure.

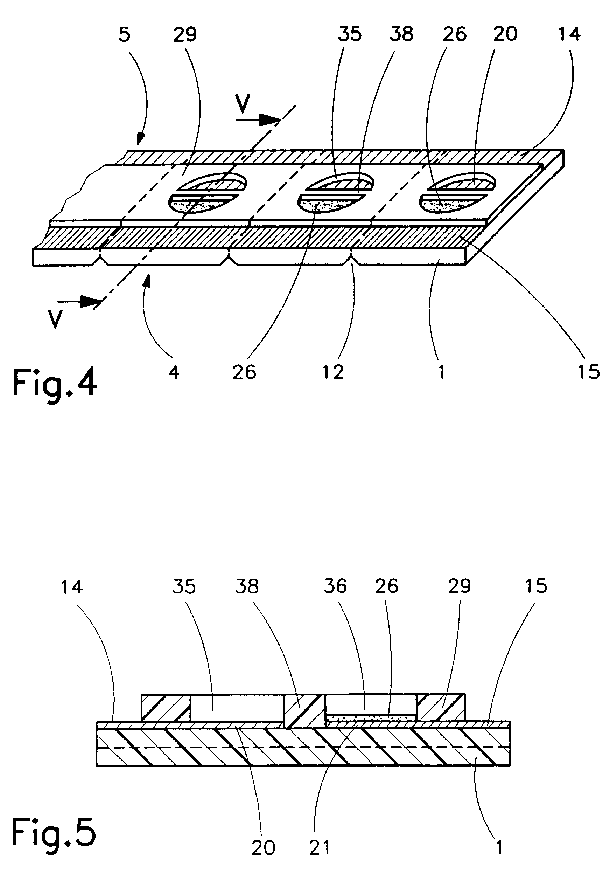

Le capteur correspondant à la variante représentée aux figures 4 et 5 comporte de la même façon une bande support 1 réalisée dans un matériau isolant cassant, tel que le mica, le verre, ou la céramique. Les électrodes 20,21 recouvrent tout ou partie de la surface de la bande 1 et sont séparées l'une de l'autre par un étroit espace médian 38 permettant de les maintenir isolées électriquement. Le film isolant de recouvrement 29 comporte uniquement des fenêtres 35;36 pour chaque zone de mesure 34, régulièrement espacées comme elles l'étaient dans le premier mode de réalisation; toutefois ce film de recouvrement 29 a une largeur inférieure à celle de la bande support 1 de sorte que des pistes conductrices 14, 15 se trouvent ménagées près de chaque bord 4, 5 pour permettre d'assurer un contact glissant avec le dispositif de connexion de l'appareil de mesure. De la même façon, les zones découvertes de l'électrode de mesure comportent le réactif 26. La bande support 1 comporte enfin, sur sa surface inférieure et perpendiculairement à l'axe médian de la bande, des rainures 12 qui permettent de positionner chaque zone de mesure et qui facilitent le cassage après usage de la zone active considérée.

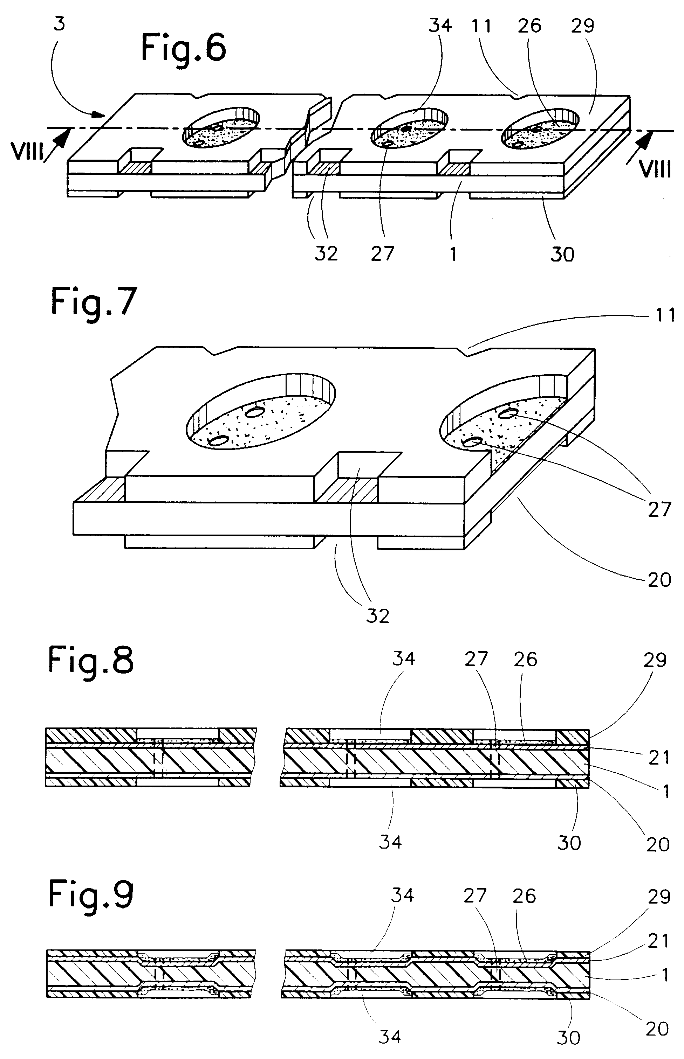

Dans la variante représentée aux figures 6 à 9, contrairement aux exemples précédents, les électrodes 20,21 sont disposées de part et d'autre de la bande support isolante 1. Un tel capteur présente donc une surface supérieure et une surface inférieure ayant sensiblement la même configuration. Les électrodes 20, 21 recouvrent respectivement tout ou partie de la surface supérieure et de la surface inférieure de la bande 1 en épousant le contour de chaque zones de mesure. Chaque face de la bande est revêtue d'un film de recouvrement laminé, collé ou sérigraphié 29, 30 comportant au droit de chaque zone de mesure une fenêtre 34 et entre deux fenêtres consécutives, des fenêtres de contact 32 près d'un bord de la bande, et de part et d'autre de celle-ci. Le réactif 26 est déposé dans les fenêtres de l'une des faces de la bande 1 comportant l'électrode de travail, le film de recouvrement 29 ayant alors avantageusement une épaisseur supérieure au film de recouvrement 30, comme représenté à la figure 8. Dans un demi-disque délimité par un axe perpendiculaire à l'axe médian de la bande et passant par le centre de chaque zone de mesure, un poinçonnage traversant 27 est effectué pour permettre le contact ionique des deux électrodes par l'échantillon à analyser lorsque la zone de mesure est prête à l'usage, comme représenté sur la figure 7. Des encoches de positionnement 11 peuvent également être prévues dans l'épaisseur des bords de la bande. Ces encoches coopèrent avec les moyens d'entraînement prévus dans l'appareil de mesure dans le but de présenter à l'utilisateur une nouvelle zone active lorsque la mesure précédente est terminée.

Selon une variante représentée par la coupe de la figure 9, la bande support isolante 1 est modelée en coupelle au droit de chaque zone de mesure par estampage ou par déformation à chaud; les films de recouvrement 20, 21 peuvent alors être d'égale épaisseur, et plus fins.

Selon une autre variante, non représentée, un capteur selon l'invention comprend toutes les caractéristiques du capteur précédemment décrit (figure 6 à 9) mais comporte des contacts ménagés à une extrémité 2 ou 3 de part et d'autre de la bande support 1.

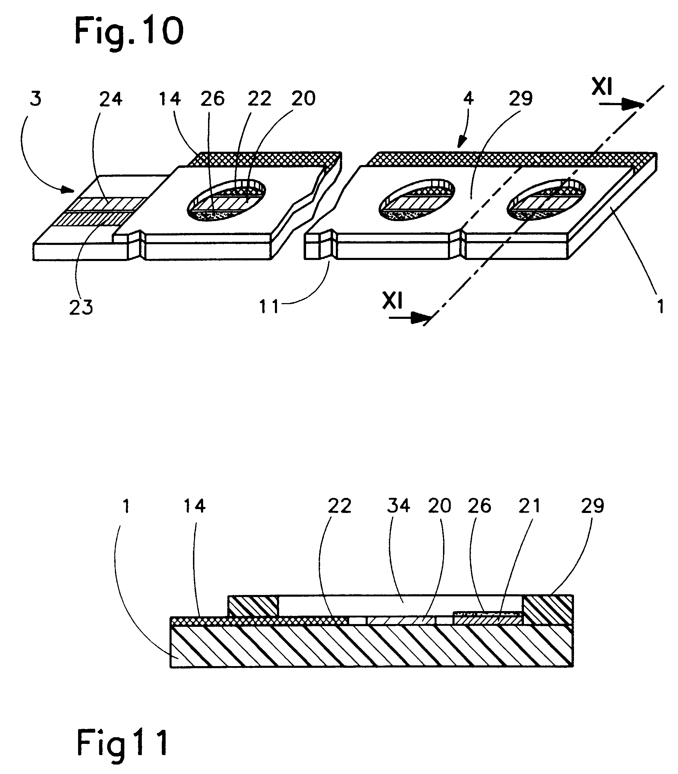

Selon un autre mode de réalisation, représenté aux figures 10 et 11, un capteur selon l'invention comporte plus de deux électrodes, par exemple une électrode de mesure, une contre électrode et une électrode de référence 20, 21, 22. Les trois électrodes sont disposées à plat sur une bande support isolante et suffisamment espacées pour assurer leur isolation électrique. La bande de recouvrement isolante 29 comporte des fenêtres 34 régulièrement espacées; cette bande a une longueur et une largeur inférieures à celles de la bande support 1 de sorte que l'extrémité 3 comporte à découvert deux portions d'électrode 20,21 et que le bord 4 comporte une piste 14 de même longueur que la bande 29 constituant ensemble trois zones de contact et de raccordement 14, 23, 24, avec un appareil de mesure approprié.

Les exemples de capteurs selon l'invention qui viennent d'être décrits sont représentés dans les figures 1 à 11 sous forme d'une barrette; lorsqu'on choisit pour la bande support isolante 1 un matériau souple, il est bien évident que ces mêmes configurations de capteurs peuvent être obtenues sous forme d'un rouleau.

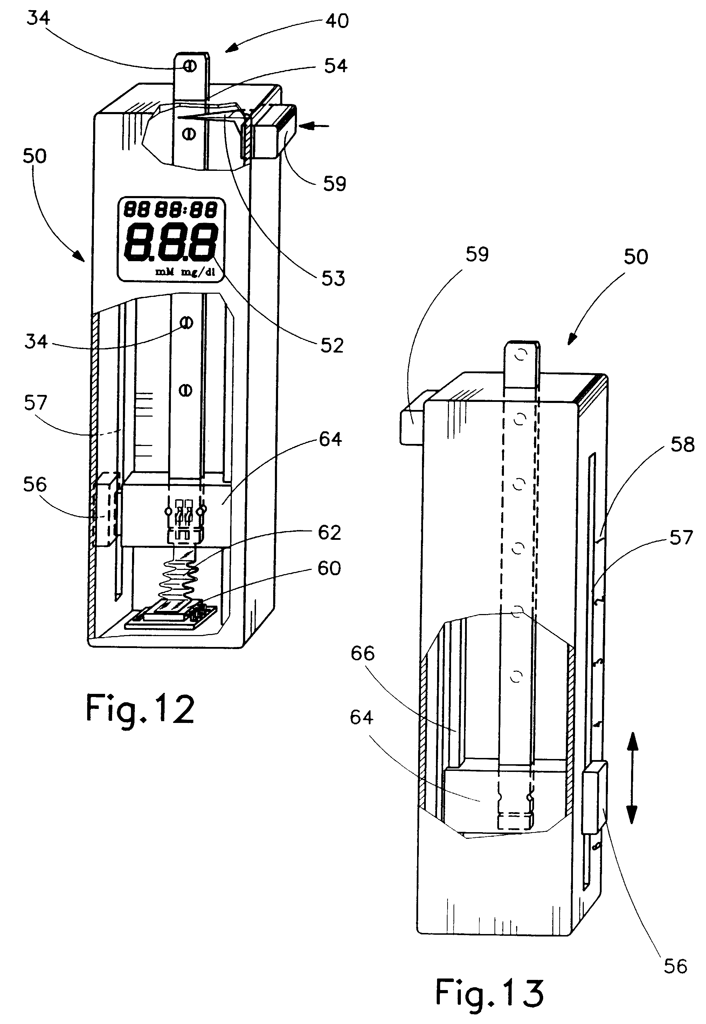

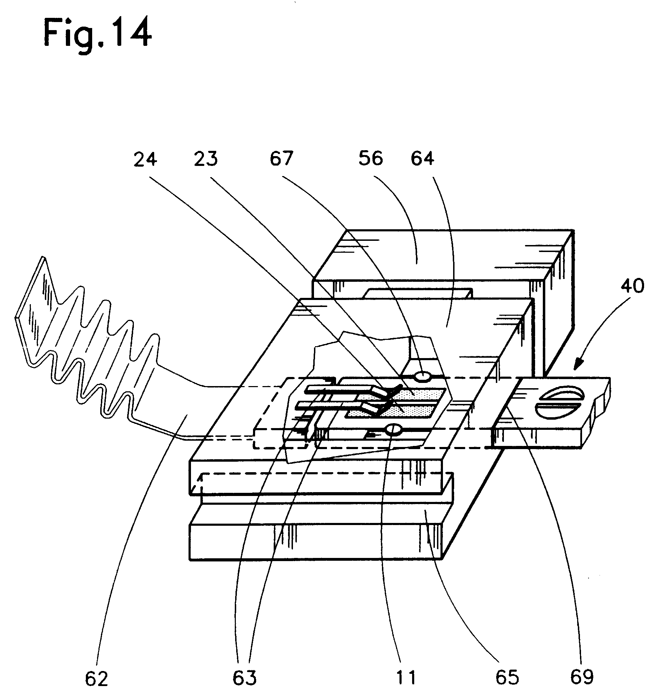

Les figures 12 à 14 illustrent un exemple de réalisation de l'appareil de mesure selon l'invention. Dans les vues de face (figure 12) et arrière (figure 13) partiellement arrachées, le boîtier 50 est représenté sous la forme d'un étui allongé ayant une longueur légèrement supérieure à celle du capteur 40 lui-même; dans cet exemple le capteur représenté est du type de celui décrit dans les figures 1 à 3. Le boîtier 50 comporte sur une face avant un dispositif d'affichage 52, par exemple à cristaux liquides à sept segments pour l'affichage des valeurs numériques. Sur l'un des côtés du boîtier un curseur 56 peut être déplacé le long d'une glissière 57, en regard d'une réglette de repérage 58 comportant des index dont l'espacement correspond à l'espacement des zones actives sur le capteur. Sur l'autre côté du boîtier, un bouton 59 prolongé à l'intérieur du boîtier par une lame tranchante 53 permet, en exerçant une pression, d'éliminer une zone du capteur utilisée. Dans sa partie supérieure le boîtier 50 compte une fente 54 dans laquelle est introduit le capteur 40 afin d'être connecté au dispositif 64. Le dispositif de connexion 64 est maintenu en place dans le boîtier 50 en étant solidaire du curseur 56 et en étant guidé par une rampe 66 permettant son déplacement à l'intérieur du boîtier; ce dispositif de connexion 64 est d'autre part relié électriquement au circuit électronique 60 au moyen d'un cordon souple 62. La figure 14 représente une vue agrandie partiellement arrachée du dispositif de connexion 64. Ce dispositif est constitué par un petit bloc comportant une fente 69 permettant d'introduire et guider le capteur 40, maintenu en place par coopération des encoches 11 et des rouleaux 67. Le contact du capteur 40 avec le circuit électronique 60 est assuré au moyen de lames élastiques 63 assujetties au cordon 62 et entrant en contact avec les zones 23, 24 du capteur au moyen d'un raccord sauté. Le dispositif 64 comporte enfin une glissière 65 venant coopérer avec la rampe 66 pour le guidage du capteur dans le boîtier.

Quels que soient les modes de réalisation, le capteur selon l'invention permet, après avoir été introduit dans l'appareil de mesure, d'effectuer avec un capteur unique plusieurs mesures avant de pourvoir à son remplacement. Le capteur est introduit dans l'appareil sur toute sa longueur, à l'exception de la première zone de mesure se trouvant à l'extrémité 2.

Pour les modes de réalisation correspondant aux figures 1, 4 et 10, la première mesure s'effectue en déposant une goutte de l'échantillon (par exemple une goutte de sang pour la mesure du taux de glucose) sur la première zone de mesure.

Pour les modes de réalisation correspondant aux figures 6 à 9 et à ses variantes, on élimine d'abord une petite portion en bout de capteur (figure 7) et on imbibe l'extrémité ainsi découverte d'une goutte de l'échantillon à analyser; la capillarité à travers les trous 27 assure un chemin de conduction ionique entre les deux électrodes.

Une fois la mesure effectuée, l'appareil de mesure permet, par un dispositif approprié, d'éliminer la zone utilisée en la coupant, ou en la cassant selon la nature de la bande support isolante 1. Les modes de réalisation représentés aux figures 1 et 9 correspondent à des exemples où la zone utilisée est éliminée de préférence par coupure; dans le mode de réalisation représenté à la figure 4 cette élimination est effectuée par cassure selon l'amorce constituée par une rainure 12. Il est bien évident que ces moyens de séparation peuvent être transposés d'un mode de réalisation à l'autre.

Pour effectuer les mesures suivantes, il suffit de manipuler les dispositifs d'avancement et de sectionnement pour mettre en place une nouvelle zone de mesure, et répéter l'opération jusqu'à la dernière zone présente sur le capteur.

## Revendications

1. Système de mesures électrochimiques constitué par un appareil de mesure (50) destiné à recevoir un dispositif capteur de petite dimension à multizones actives de mesure (40), séquentiellement jetables, permettant d'effectuer au moyen d'un unique capteur introduit dans l'appareil plusieurs mesures successives d'une même caractéristique physicochimique, ou d'une concentration d'un même composant présent dans une solution, un effluent, ou un fluide d'origine naturelle, biologique ou biochimique, l'appareil de mesure comportant :
- un boîtier (50),
- un circuit électronique (60) et une source d'énergie situés à l'intérieur dudit boîtier, destinés d'une part à imposer un courant électrique variable ou non, ou une différence de potentiel variable ou non, entre les contacts recevant le capteur, d'autre part à traiter le signal électrique résultant et à afficher la valeur représentative de la caractéristique mesurée.
- au moins une ouverture (54) pratiquée dans le boîtier destinée à recevoir un capteur d'une longueur déterminée.
- un dispositif (64) permettant de connecter électriquement le capteur (40) introduit dans ledit boîtier au circuit électronique (60) pendant toute la durée d'une série de mesures.
- un dispositif (56, 57, 58) permettant de positionner et de déplacer le capteur à l'intérieur du boîtier.
- un dispositif (59, 53) permettant d'éliminer une zone de mesure du capteur après chaque mesure
caractérisé en ce que le dispositif capteur comporte
- un support mince isolant (1) en forme de bande
- au moins deux collecteurs (16, 17) de courant en matériaux conducteurs disposés en forme de bandes ou de films sur ledit support isolant, suffisamment proches l'un de l'autre mais électriquement isolés.
- un ou plusieurs revêtements (29, 30) isolants recouvrant partiellement la bande et les collecteurs de courant (16, 17) en délimitant au moins une zone de contact (23, 24) avec le circuit électronique de mesure (60) et en comportant au moins deux fenêtres (34) consécutives traversées par les collecteurs de courant formant électrodes (20, 21), et constituant avec la bande support les zones actives de mesure.

2. Système de mesure selon la revendication 1, caractérisé en ce que les collecteurs de courant (16, 17) du capteur sont situées sur une même face de la bande support isolante (1).

3. Système de mesure selon la revendication 1, caractérisé en ce que les collecteurs de courant (16, 17) du capteur sont situées sur les faces opposées de la bande support isolante (1).

4. Système de mesure selon les revendications 1, 2 ou 3, caractérisé en ce que les zones de contact (23, 24) du capteur avec un appareil de mesure (50) sont situées aux extrémités des collecteurs de courant (16, 17).

5. Système de mesure selon les revendications 1, 2 ou 3, caractérisé en ce que les zones de contact (14, 15) du capteur avec un appareil de mesure sont situées le long des bords externes des collecteurs de courant (16, 17).

6. Système de mesure selon les revendications 4 ou 5, caractérisé en ce que les zones de contact (14, 15, 23 ou 24) du capteur sont situées dans des fenêtres ménagées dans la bande de recouvrement.

7. Système de mesure selon les revendications 2 ou 3, caractérisé en ce que les collecteurs de courant (16, 17) du capteur sont constitués par des bandes obtenues par déposition en couche mince ou épaisse de matériaux conducteurs.

8. Système de mesure selon les revendications 2 ou 3, caractérisé en ce que les collecteurs de courant (16, 17) du capteur sont constitués par des films isolants métallisés au moyen d'un matériau conducteur, et laminés sur le support mince isolant.

9. Système de mesure selon les revendications 7 ou 8, caractérisé en ce que le matériau conducteur est l'or, l'argent, le platine, le nickel, le palladium, le titane ou le carbone.

10. Système de mesure selon les revendications 1, 2 ou 3, caractérisé en ce que la bande support isolante (1) du capteur est constituée par un matériau souple configuré en forme de barrette ou de rouleau.

11. Système de mesure selon la revendication 10, caractérisé en ce que le matériau souple est un polyéthylène téréphtalate.

12. Système de mesure selon les revendications 1, 2 ou 3, caractérisé en ce que la bande support isolante (1) du capteur est constituée par un matériau cassant.

13. Système de mesure selon la revendication 12, caractérisé en ce que le matériau cassant est du mica, du verre ou de la céramique.

14. Système de mesure selon les revendications 1, 2 ou 3, caractérisé en ce que la bande support isolante du capteur comporte en outre des moyens de positionnement constitués par des encoches (11) régulièrement espacées dans l'épaisseur d'au moins un bord de la bande.

15. Système de mesure selon les revendications 1 et 2, caractérisé en ce que la bande support isolante (1) du capteur comporte en outre des moyens de positionnement et de sectionnement constitués par des rainures (12) régulièrement espacées dans la surface de la bande opposée à la surface comportant les zones de mesure.

16. Système de mesure selon les revendications 1 et 3, caractérisé en ce que les revêtement de recouvrement (29, 30) de la bande support isolante du capteur et des collecteurs de courant sont d'épaisseurs inégales de part et d'autre de la bande support.

17. Système de mesure selon la revendication 16, caractérisé en ce que les fenêtres (34) pratiquées dans la bande de recouvrement ayant la plus grande épaisseur délimitent avec la bande support isolante une cavité susceptible de recevoir un réactif (26).

18. Système de mesure selon les revendications 1 et 3, caractérisé en ce que le fond des cavités délimitées par la bande support isolante (1) du capteur et les fenêtres pratiquées (34) dans les bandes de recouvrement (29, 30) est mis en forme de coupelle par estampage ou déformation à chaud.

19. Système de mesure selon les revendications 1 à 17, caractérisé en ce qu'un collecteur de courant (16) du capteur est en platine et constitue une électrode de travail (21) et que l'autre collecteur de courant (17) est en argent/chlorure d'argent et constitue une électrode de référence (20).

20. Système de mesure selon la revendication 19, caractérisé en ce que l'électrode de travail (21) est en outre revêtue d'un réactif (26) comportant au moins une enzyme spécifique du composant présent dans la solution.

21. Système de mesure selon la revendication 20, caractérisé en ce que le réactif (26) comporte en outre un médiateur.

22. Système de mesure selon la revendication 21, caractérisé en ce que le médiateur est choisi parmi les complexes d'un métal de transition avec au moins un ligand bipyridine, terpyridine ou phenantroline substitué par au moins un groupe donneur d'électrons.

23. Système de mesure selon les revendications 19 à 22, caractérisé en ce que l'enzyme spécifique est la glucose oxydase pour effectuer le dosage du glucose.

24. Système de mesure selon la revendication 1, caractérisé en ce que la section transversale sensiblement rectangulaire du capteur présente une plus grande dimension comprise entre 6 et 12 mm, et une plus petite dimension comprise entre 0,05 et 0,5 mm.

25. Appareil de mesure selon la revendication 1, caractérisé en ce que le dispositif de positionnement et d'avancement comporte un organe de commande (56) situé sur une partie extérieure du boîtier et prolongé à l'intérieur du boîtier par un mécanisme (64) venant coopérer avec les encoches (11) ou les rainures (12) de la bande support du capteur.

26. Appareil de mesure selon la revendication 25, caractérisé en ce qu'une seule manipulation de l'organe de commande (56) provoque le déplacement du capteur d'une longueur égale au pas nécessaire pour effectuer la mesure suivante.

27. Appareil de mesure selon la revendication 1, caractérisé en ce que le dispositif d'élimination (59) d'une zone de mesure utilisée est constitué par une lame tranchante (53) coopérant avec un dispositif mettant en contact la lame et le capteur, ledit dispositif étant assujetti à un organe de commande situé sur une partie extérieure du boîtier et indépendant de l'organe de commande d'avancement de la bande support ou assujetti à celui-ci.

28. Appareil de mesure selon la revendication 1, caractérisé en ce que le circuit électronique (60) comprend en outre une base de temps destinée à délivrer un signal correspondant à la fréquence souhaitée pour les mesures.

29. Appareil de mesure selon la revendication 1, caractérisé en ce que le circuit électronique (60) comprend en outre un thermomètre de compensation de la température.

## Patentansprüche

1. Elektrochemisches Meßsystem mit einem Meßgerät (50) zur Aufnahme eines Sensorelements (40) kleiner Abmessung mit mehreren aktiven Meßzonen, die sequentiell ausfahrbar sind, wodurch mittels eines einzigen in das Gerät eingeführten Sensorelements mehrere aufeinanderfolgende Messungen derselben physikalisch-chemischen Eigenschaft oder eine in einer Lösung, einem Abwasser oder einer Flüssigkeit natürlichen Ursprungs vorhandene Konzentration derselben Komponente durchführbar sind, wobei das Meßgerät
- ein Gehäuse (50),
- eine elektronische Schaltung (60) und eine elektrische Energiequelle, die im Inneren des Gehäuses angeordnet sind, und die einerseits zum Aufprägen eines variablen oder nicht variablen elektrischen Stroms oder einer variablen oder nicht variablen Potentialdifferenz zwischen den das Sensorelement aufnehmenden Kontakten und andererseits zur Verarbeitung des resultierenden elektrischen Signals und zur Darstellung des die gemessene Eigenschaft repräsentierenden Werts bestimmt sind,
- mindestens eine öffnung (54), die in dem Gehäuse vorgesehen ist, zur Aufnahme eines Sensorelements bestimmter Länge,
- eine Vorrichtung (64), die eine elektrische Verbindung des in das Gehäuse eingeführten Sensorelements (40) mit der elektronischen Schaltung (60) während der Dauer einer Reihe von Messungen ermöglicht,
- eine Vorrichtung (56, 57, 58) zur Positionierung und zur Verschiebung des Sensorelements im Inneren des Gehäuses,
- eine Vorrichtung (59, 53) zum Entfernen einer Meßzone des Sensorelements nach jeder Messung umfaßt,
dadurch gekennzeichnet, daß das Sensorelement
- einen streifenförmigen dünnen isolierenden Träger (1),
- mindestens zwei Stromkollektoren (16, 17) aus leitenden Materalien, die streifen- oder schichtförmig auf dem isolierenden Träger angeordnet und ausreichend nahe beieinander, jedoch elektrisch isoliert sind,
- eine oder mehrere isolierende Abdeckungen (29, 30), die den Streifen und die Stromkollektoren (16, 17) teilweise abdecken, wobei sie mindestens eine Kontaktzone (23, 24) mit der elektronischen Meßschaltung (60) abgrenzen und mindestens zwei aufeinanderfolgende, von den Elektroden bildenden Stromkollektoren durchquerte Fenster (34) umfassen, und die mit dem Streifenträger die aktiven Meßzonen bilden.

2. Meßsystem nach Anspruch 1, dadurch gekennzeichnet, daß die Stromkollektoren (16, 17) des Sensorelements auf derselben Seite des streifenförmigen isolierenden Trägers (1) angeordnet sind.

3. Meßsystem nach Anspruch 1, dadurch gekennzeichnet, daß die Stromkollektoren (16, 17) des Sensorelements auf gegenüberliegenden Seiten des streifenförmigen isolierenden Trägers (1) angeordnet sind.

4. Meßsystem nach einem der Ansprüche 1 bis 3, dadurch gekennzeichnet, daß die Kontaktzonen (23, 24) des Sensorelements mit einer Meßvorrichtung (50) an den Enden der Stromkollektoren (16, 17) angeordnet sind.

5. Meßsystem nach einem der Ansprüche 1 bis 3, dadurch gekennzeichnet, daß die Kontaktzonen (14, 15) des Sensorelements mit einer Meßvorrichtung längs der äußeren Ränder der Stromkollektoren (16, 17) angeordnet sind.

6. Meßsystem nach Anspruch 4 oder 5, dadurch gekennzeichnet, daß die Kontaktzonen (14, 15, 23 oder 24) des Sensorelements in den im Streifen der Abdeckung angeordneten Fenstern vorgesehen sind.

7. Meßsystem nach Anspruch 2 oder 3, dadurch gekennzeichnet, daß die Stromkollektoren (16, 17) des Sensorelements durch Streifen gebildet werden, die durch Deposition einer dünnen oder dicken Schicht aus leitenden Materialien erhalten wurden.

8. Meßsystem nach Anspruch 2 oder 3, dadurch gekennzeichnet, daß die Stromkollektoren (16, 17) des Sensorelements durch isolierende Filme gebildet werden, die mittels eines leitendem Materials metallisiert und auf den dünnen isolierenden Träger laminiert sind.

9. Meßsystem nach Anspruch 7 oder 8, dadurch gekennzeichnet, daß das leitende Material Gold, Silber, Platin, Nickel, Palladium, Titan oder Kohlenstoff ist.

10. Meßsystem nach einem der Ansprüche 1 bis 3, dadurch gekennzeichnet, daß der streifenförmige isolierende Träger (1) des Sensorelements durch ein weiches Material gebildet wird, welches steg- oder zylinderförmig konfiguriert ist.

11. Meßsystem nach Anspruch 10, dadurch gekennzeichnet, das das flexible Material ein Polyethylenteraphthalat ist.

12. Meßsystem nach einem der Ansprüche 1 bis 3, dadurch gekennzeichnet, daß der streifenförmige isolierende Träger (1) des Sensorelements aus einem spröden Material besteht.

13. Meßsystem nach Anspruch 12, dadurch gekennzeichnet, daß das spröde Material Glimmer, Glas oder Keramik ist.

14. Meßsystem nach einem der Ansprüche 1 bis 3, dadurch gekennzeichnet, daß der streifenförmige isolierende Träger des Sensorelements außerdem Positionierungsmittel umfaßt, die aus Kerben (11) bestehen, welche in der Dicke von mindestens einem Rand des Streifens regelmäßig beabstandet sind.

15. Meßsystem nach Anspruch 1 oder 2, dadurch gekennzeichnet, daß der steifenförmige isolierende Träger (1) des Sensorelements ferner Positionierungs- und Unterteilungsmittel umfaßt, die aus Rillen (12) bestehen, welche in der Oberfläche des Streifens regelmäßig beabstandet sind, der gegenüberliegend zu der die Meßzonen umfassenden Oberfläche ist.

16. Meßsystem nach Ansprüche 1 und 3, dadurch gekennzeichnet, daß die Auflagen der Abdeckung (29, 30) des steifenförmigen isolierenden Trägers des Sensorelements und der Stromkollektoren auf beiden Seiten des streifenförmigen Trägers unterschiedliche Dicken aufweisen.

17. Meßsystem nach Anspruch 16, dadurch gekennzeichnet, daß die in dem Streifen der Abdeckung vorgesehenen Fenster (34), welche eine größere Dicke aufweisen, mit dem streifenförmigen isolierenden Träger einen Hohlraum zur Aufnahme einer Reagenz (26) abgrenzen.

18. Meßsystem nach Ansprüche 1 und 3, dadurch gekennzeichnet, daß der Boden des durch den streifenförmigen isolierenden Träger (1) des Sensorelements und die Streifen der Abdeckung (29, 30) abgegrenzten Hohlräume zum Warmpressen oder -verformen ausgebildet ist.

19. Meßsystem nach Ansprüche 1 bis 17, dadurch gekennzeichnet, daß ein Stromkollektor (16) des Sensorelements aus Platin besteht und eine Arbeitselektrode (21) darstellt und der andere Stromkollektor (17) aus Silber/Silberchlorid besteht und eine Referenzelektrode (20) darstellt.

20. Meßsystem nach Anspruch 19, dadurch gekennzeichnet, daß die Arbeitselektrode (21) ferner mit einer Reagenz (26) beschichtet ist, die mindestens ein spezifisches Enzym der in der Lösung vorliegenden Komponente umfaßt.

21. Meßsystem nach Anspruch 20, dadurch gekennzeichnet, daß die Reagenz (26) ferner einen Beschleuniger umfaßt.

22. Meßsystem nach Anspruch 21, dadurch gekennzeichnet, daß der Beschleuniger aus Komplexen eines übergangsmetalls mit mindestens einem Bipyridin, Terpyridin oder Phenantrolinliganden ausgewählt wird, welcher mit mindestens einer Donatorgruppe substituiert ist.

23. Meßsystem nach einem der Ansprüche 19 bis 22, dadurch gekennzeichnet, daß das spezifische Enzym Glucoseoxydase zur Dosierung von Glucose ist.

24. Meßsystem nach Anspruch 1, dadurch gekennzeichnet, daß der im wesentlichen rechteckige Querabschnitt des Sensorelements eine größere Abmessung zwischen 6 und 12 mm und eine kleinere Abmessung zwischen 0,05 und 0,5 mm aufweist.

25. Meßgerät nach Anspruch 1, dadurch gekennzeichnet, daß die Vorrichtung zur Positionierung und zum Vorrücken ein Steuerorgan (56) umfaßt, welches auf einem äußeren Abschnitt des Gehäuses angeordnet ist und zum Inneren des Gehäuses über einen mit den Kerben (11) oder den Rillen(12) des streifenförmigen Trägers des Sensorelements kooperierenden Mechanismus (64) verlängert ist.

26. Meßgerät nach Anspruch 25, dadurch gekennzeichnet, daß eine einzige Betätigung des Steuerorgans (56) die Verschiebung des Sensorelements um eine Länge hervorruft, die gleich dem zur Durchführung der nachfolgenden Messung erforderlichen Schritt ist.

27. Meßgerät nach Anspruch 1, dadurch gekennzeichnet, daß die Vorrichtung zum Entfernen (59) einer benutzten Meßzone aus einer scharfen Klinge (53) gebildet ist, die mit einer Vorrichtung kooperiert, welche die Klinge und das Sensorelement in Kontakt bringt, wobei die Vorrichtung einem Steuerorgan unterworfen ist, welches sich in einem äußeren Abschnitt des Gehäuses befindet und unabhängig von dem Steuerorgan zum Vorrücken des streifenförmigen Trägers oder diesem unterworfen ist.

28. Meßgerät nach Anspruch 1, dadurch gekennzeichnet, daß die elektronische Schaltung (60) ferner eine Zeitbasis umfaßt, die zur Abgabe eines Signals entsprechend der gewünschten Frequenz für die Messungen bestimmt ist.

29. Meßgerät nach Anspruch 1, dadurch gekennzeichnet, daß die elektronische Schaltung (60) ferner ein Thermometer zur Kompensation der Temperatur umfaßt.

## Claims

1. Electrochemical measuring system composed of a measuring apparatus (50) adapted to receive a small sensor device with active sequentially disposable measuring multizones (40) which make it possible to carry out using a single sensor introduced into the apparatus several successive measurements of the same physicochemical characteristic or of a concentration of the same component present in a solution, an effluent or a fluid of natural, biological or biochemical origin, the measuring apparatus having :
- a housing (50),
- an electronic circuit (60) and an energy source situated inside said housing intended, on the one hand, to impose a variable or non-variable electric current or a variable or non-variable potential difference between the contacts receiving the sensor, on the other hand, to treat the resulting electrical signal and to display the value representative of the characteristic measured,
- at least one opening (54) provided in the housing adapted to receive a sensor of a determined length,
- a device (64) which makes it possible to connect the sensor (40) introduced in said housing electrically to the electronic circuit (60) throughout the duration of a series of measurements,
- a device (56, 57, 58) making it possible to position and move the sensor inside the housing,
- a device (59, 53) which makes it possible to remove one measuring zone of the sensor after each measurement,
characterized in that the sensor device has
- a thin insulating support (1) in the form of a strip,
- at least two current collectors (16, 17) of conducting material arranged on said insulating support in the form of strips or films sufficiently close to each other, but electrically insulated,
- one or several insulating coverings (29, 30) partially covering the strip and the current collectors (16, 17) by delimiting at least one contact zone (23, 24) with the measuring electronic circuit and by having at least two consecutive windows (34) traversed by the current collectors constituting electrodes (20, 21) and constituting the active measuring zones together with the support strip.

2. Measuring system according to claim 1, characterized in that the current collectors (16, 17) of the sensor are situated on the same side of the insulating support strip (1).

3. Measuring system according to claim 1, characterized in that the current collectors (16, 17) of the sensor are situated on opposite sides of the insulating support strip (1).

4. Measuring system according to claims 1, 2 or 3, characterized in that the contact zones (23, 24) of the sensor with a measuring apparatus (50) are situated at the extremities of the current collectors (16, 17).

5. Measuring system according to claims 1, 2 or 3, characterized in that the contact zones (14, 15) of the sensor with a measuring apparatus are located along the outside edges of the current collectors (16, 17).

6. Measuring system according to claims 4 or 5, characterized in that the contact zones (14, 15, 23 or 24) of the sensor are situated in windows provided in the covering strip.

7. Measuring system according to claims 2 or 3, characterized in that the current collectors (16, 17) of the sensor are composed of strips obtained by thin-layer or thick-layer deposition of conducting materials.

8. Measuring system according to claims 2 or 3, characterized in that the current collectors (16, 17) of the sensor are composed of insulating films metallised using a conducting material and laminated on the thin insulating support.

9. Measuring system according to claims 7 or 8, characterized in that the conducting material is gold, silver, platinum, nickel, palladium, titanium or carbon.

10. Measuring system according to claims 1, 2 or 3, characterized in that the insulating support strip (1) of the sensor is composed of a flexible material configured in the form of a bar or a roll.

11. Measuring system according to claim 10, characterized in that the flexible material is a polyethylene terephthalate.

12. Measuring system according to claims 1, 2 or 3, characterized in that the insulating support strip (1) of the sensor is composed of a brittle material.

13. Measuring system according to claim 12, characterized in that the brittle material is mica, glass or ceramic.

14. Measuring system according to claims 1, 2 or 3, characterized in that the insulating support strip of the sensor also has positioning means composed of notches (11) regularly spaced in the thickness of at least one edge of the strip.

15. Measuring system according to claims 1 and 2, characterized in that the insulating support strip (1) of the sensor also has positioning means and sectioning means composed of grooves (12) regularly spaced in the surface of the strip opposite the surface having the measuring zones.

16. Measuring system according to claims 1 and 3, characterized in that the covering coatings (29, 30) of the insulating support strip of the sensor and the current collectors are of unequal thickness on both sides of the support strip.

17. Measuring system according to claim 16, characterized in that the windows (34) provided in the covering strip having the greatest thickness cooperate with the insulating support strip to delimit a cavity capable of receiving a reagent (26).

18. Measuring system according to claims 1 and 3, characterized in that the base of the cavities delimited by the insulating support strip (1) of the sensor and the windows (34) provided in the covering strips (29, 30) acquire a cup shape by stamping or heat distortion.

19. Measuring system according to claims 1 to 17, characterized in that one current collector (16) of the sensor is of platinum and constitutes a working electrode (21) and that the other current collector (17) is of silver/silver chloride and constitutes a reference electrode (20).

20. Measuring system according to claim 19, characterized in that the working electrode (21) is also covered by a reagent (26) having at least one specific enzyme of the constituent present in the solution.

21. Measuring system according to claim 20, characterized in that the reagent (26) also has a mediator.

22. Measuring system according to claim 21, characterized in that the mediator is chosen from amongst the complexes of a transition metal with at least one bipyridine, terpyridine or phenantroline ligand substituted by at least one group of electron donors.

23. Measuring system according to claims 19 to 22, characterized in that the specific enzyme is glucose oxidase to effect the quantitative analysis of glucose.

24. Measuring system according to claim 1, characterized in that the transverse, substantially rectangular section of the sensor has one larger dimension comprising between 6 and 12 mm, and one smaller dimension comprising between 0.05 and 0.5 mm.

25. Measuring apparatus according to claim 1, characterized in that the positioning and advancing device has control means (56) located on an outside part of the housing and continued inside the housing by a mechanism (64) that cooperates with notches (11) or grooves (12) of the support strip of the sensor.

26. Measuring apparatus according to claim 25 , characterized in that a single manipulation of the control means (56) causes the displacement of the sensor of an equal length to the extent necessary to effect the subsequent measurement.

27. Measuring apparatus according to claim 1, characterized in that the eliminating device (59) of a used measuring zone used is composed of a cutting blade (53) cooperating with a device bringing the blade into contact with the sensor, said device being fixed to a control organ located on an outer part of the housing and independent of the control organ for advancing the support strip or fixed thereto.

28. Measuring apparatus according to claim 1, characterized in that the electronic circuit (60) also comprises a time base adapted to deliver a signal corresponding to the frequency required for the measurements.

29. Measuring apparatus according to claim 1, characterized in that the electronic circuit (60) also comprises a thermometer for temperature compensation.
